# EUROPEAN PATENT APPLICATION

(11) **EP 3 476 425 A1**
(43) Date of publication of application: **01.05.2019**
(21) Application number: 17306502.0
(22) Date of filing: 31.10.2017
(51) Int. Cl.: A61M 25/02

(54) **MEDICAL DEVICE TO SECURE A CATHETER TO A PATIENT'S SKIN**

(71) Applicant: Centre Hospitalier Universitaire de Nice, 06200 Nice (FR)
(72) Inventor: SEJOR, Eric, 06000 Nice (FR)
(74) Representative: Osha Liang

(57) **Abstract**

A medical device (100) to secure a catheter to a patient's skin comprising: a pair of upper jaws comprising a left upper jaw (101) and a right upper jaw (102) adapted to receive a catheter there between; a pair of lower jaws comprising a left lower jaw (103) and a right lower jaw (104) adapted to receive the catheter there between; a pair of guiding rods (105, 106) connecting the left and right upper jaws (101, 102), and guiding the left and right upper jaws (101, 102) in translation relative to each other; and a first pair of inclined rods (107) connecting the left upper jaw (101) to the right lower jaw (104) and a second pair of inclined rods (108) connecting the right upper jaw (102) to the left lower jaw (103) so that the pair of lower jaws (103, 104) closes when the pair of upper jaws (101, 102) opens and the pair of lower jaws (103, 104) opens when the pair of upper jaws (101, 102) closes.

## Description

### TECHNICAL FIELD

The present disclosure relates generally to a medical device for securing a catheter to a patient's skin.

### BACKGROUND

It is commonly known to use catheters in the treatment and care of patients. Catheters are hollow tubes used to allow passage of fluid from or into a patient's body. In particular, they can be inserted into canals, vessels, passageways, or body cavities to permit injection or withdrawal of fluids or to keep a passage open. Following its insertion into the patient's body, the catheter is often secured to the patient. The catheter has to be secured to the patient in a manner that minimizes the movement of the catheter relative to the patient's body, because such a movement could harm the patient or otherwise interrupt proper functioning of the catheter.

One approach in the prior art is to tie the catheter to the patient's skin by means of a suture thread. See patent document US 2013/0338599. However, the suture thread may break. Moreover, when the catheter has to be moved (or mobilized), the suture makes the mobility of the catheter difficult and it may be necessary to cut the suture thread and repeat the suture.

Alternatively, the catheter is secured to the patient's skin by using an adhesive tape. See patent document US5807341A. However, the use of an adhesive tape can be undesirable due to the covering of the catheter insertion site. This can make it hard to identify infections, drainage or other complications that may occur at the catheter insertion site.

Thus, there still exists a need for new alternatives to secure a catheter to a patient's skin.

### GENERAL PRESENTATION

According to one aspect of the present disclosure, a medical device to secure a catheter to a patient's skin is provided. Such a device comprises a pair of upper jaws and a pair of lower jaws. The pair of upper jaws comprises a left upper jaw and a right upper jaw adapted to receive a catheter therebetween. The pair of lower jaws comprises a left lower jaw and a right lower jaw adapted to receive the catheter therebetween. The device also comprises a pair of guiding rods connecting the left and right upper jaws, and guiding the left and right upper jaws in translation relative to each other. The device further comprises a first pair of inclined rods connecting the left upper jaw to the right lower jaw and a second pair of inclined rods connecting the right upper jaw to the left lower jaw, so that the pair of lower jaws closes when the pair of upper jaws opens and the pair of lower jaws opens when the pair of upper jaws closes.

For securing a catheter to a patient's skin with such a medical device, the catheter is arranged between the pair of upper jaws and between the pair of lower jaws. Then, the pair of lower jaws is closed so that the lower jaws clamp the catheter while the pair of upper jaws opens. Afterwards, the catheter and the pair of lower jaws, is inserted into the patient's body, through the patient's skin, until the pair of upper jaws abuts against the outer surface of the patient's skin. Finally, the pair of upper jaws is closed so that the upper jaws clamp the catheter while the pair of lower jaws opens, thereby fixing the medical device and catheter in position. When the lower jaws open, they enter the patient's tissues and, thus, anchor the medical device in the patient's body.

Optionally, the catheter may be mobilized (i.e. moved relative to the patient's body) by: partially opening the pair of upper jaws, so that the pair of upper jaws do not clamp the catheter anymore and the lower jaws do not clamp the catheter either; moving the catheter relative to the medical device; and closing the pair of upper jaws so that the upper jaws clamp the catheter and the lower jaws open. Thus, the catheter may be mobilized without removing the medical device out of the patient's body.

Such a medical device makes it possible to secure a catheter to a patient's skin quite easily and rapidly in a reproducible and effective manner. Such a device is disposable or reusable after sterilization. Compared to the existing technique using a suture thread, the medical device makes it possible to mobilize the catheter more easily, more frequently and rapidly, with less pain for the patient. Furthermore, compared to an adhesive tape, the medical device allows the catheter insertion site not to be covered. This ensures a lower risk of infections or other complications that may occur at the catheter insertion site.

In the present disclosure, the term "catheter" encompasses any kind of pipe or tube such as flexible tube or rigid tube. In particular the term encompasses the drain, drain catheter or catheter. Also, the terms "patient's skin" encompasses any kind of skin, being animal or human tissues.

In certain embodiments, the pair of upper jaws and lower jaws respectively form upper and lower rings when they are closed, each jaw having a half-circle shape. The inner diameter of the upper ring may be similar to the diameter of the lower ring. Thus, the circular shape of the upper and lower rings is complementary with the outer cross-section of the catheter which has, usually, a constant circular cross-section. Typically, the inner diameter of the upper and lower rings is adapted or adaptable to the diameter of the catheter.

In certain embodiments, the inclined rods of the first pair of inclined rods are parallel and the inclined rods of the second pair of inclined rods are parallel. The guiding rods of the pair of guiding rods may also be parallel. These rods are sufficiently spaced apart from each other to allow the catheter to pass between them.

In certain embodiments, the pair of guiding rods is provided with at least one spring which pushes the right upper jaw away from the left upper jaw. When no external force is applied by an operator on the pair of upper jaws, the spring maintains the right upper jaw away from the left upper jaw. As a consequence, the right and left lower jaws are maintained in their closed position.

In certain embodiments, the medical device comprises a locking system for locking the pair of upper jaws in their closed position. Consequently, the locking system locks the pair of lower jaws in their opened position, thereby fixing the medical device and catheter in position relative to the patient's skin.

In certain embodiments, the locking mechanism comprises at least one hook provided on one of the upper jaws and a locking notch provided on the other upper jaw. The hook interlocks with the locking notch when the pair of upper jaws is closed. The locking mechanism may comprise a locking or unlocking button connected to the hook or the locking notch. Such a locking or unlocking button facilitates the use of the locking mechanism.

In certain embodiments, the guiding rods go through the upper jaws and the ends of the guiding rods are provided with a stopper such that the relative movement between a guiding rod and an upper jaw is limited when the jaw abuts against the stopper. In other terms, the guiding rods are engaged with the upper jaws and cannot disengage from the upper jaws because of the stoppers.

In other embodiments, each guiding rod is telescopic, i.e. it comprises concentric tubular sections designed to slide into one another. The length of such a guiding rod decreases when the upper jaws close and increases when the upper jaws open.

In certain embodiments, the upper jaws and/or the lower jaws define an inner contact surface intended to be in contact with the catheter when the upper jaws and/or the lower jaws close, the inner contact surface being provided with at least one protrusion for gripping the catheter when the upper jaws and/or the lower jaws close. Thus, the catheter is clamped more efficiently. For instance, both the upper and lower jaws define an inner contact surface intended to be in contact with the catheter when the upper and lower jaws close, respectively, and at least one of the inner contact surfaces may be provided with one or several protrusions, for instance one or several teeth, for gripping the outer surface of the catheter.

In certain embodiments, two press buttons are respectively provided on the upper jaws in diametrically opposed positions. The press buttons may form large surfaces on which a pressure can be exerted easily, for instance with the thumb and the index finger, in order to close the pair of upper jaws.

According to another aspect of the present disclosure, there is provided a method for securing a catheter to a patient's skin using the medical device of the present description.

In certain embodiments, the method comprises the steps of: arranging the catheter between the pair of lower jaws and between the pair of upper jaws; closing the pair of lower jaws so that the lower jaws clamp the catheter while the pair of upper jaws opens; inserting the catheter and the pair of lower jaws into the patient's body, through the patient's skin, until the pair of upper jaws are close to or abuts against the outer surface of the patient's skin; and closing the pair of upper jaws so that the upper jaws clamp the catheter while the pair of lower jaws opens, thereby fixing the medical device and catheter in position.

For mobilizing a catheter, the method may further comprise the steps of: partially (i.e. not fully) opening the pair of upper jaws so that the upper jaws do not clamp the catheter anymore and the lower jaws do not clamp the catheter either (the lower jaws would clamp the catheter if the upper jaws were fully opened); moving the catheter relative to the medical device; closing the pair of upper jaws so that the upper jaws clamp the catheter and the lower jaws open.

Such a method has the advantages derived from using a medical device according to the present disclosure. Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same or like parts throughout the different views. The drawings are not necessarily to scale, emphasis instead being placed upon illustrating the principles of the invention.
FIGS 1A to 1C are perspective views of an example of a medical device to secure a catheter to a patient's skin, shown in three different positions.
FIGS 2A and 2B illustrate in details the locking system of the medical device of FIG 1A.
FIGS 3A and 3B are diagrams illustrating two steps of a method for securing a catheter to a patient's skin, using a medical device such as that of FIG 1A.

### DETAILED DESCRIPTION

The accompanying drawings showing an example of a medical device to secure a catheter to a patient's skin are referred to in the following detailed description. It is intended that this example be considered as illustrative only, the invention not being limited to this example.

To avoid unnecessary details for practicing the invention, the description may omit certain information already known to those skilled in the art.

The words left, right, upper, lower and the like refer to the orientation shown in the appended drawings, as well as the normal connotations of these words when applied to the medical device described. However, they are not intended to restrict the positioning of the medical device in actual use.

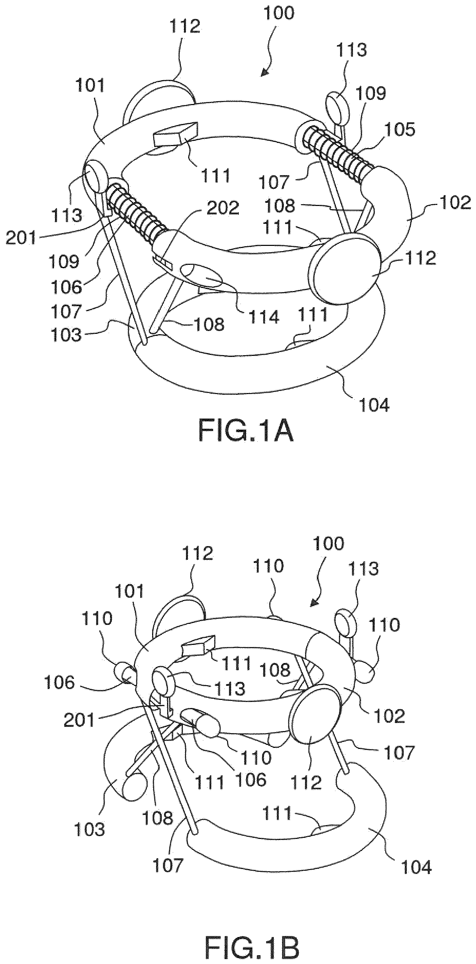

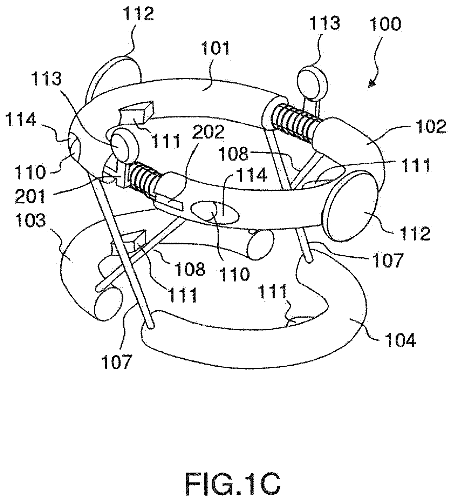

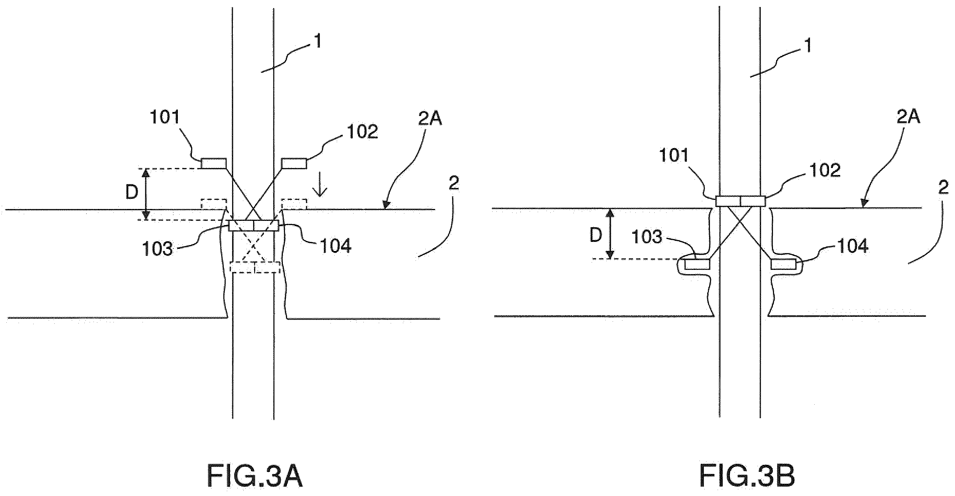

FIGS 1A to 1C illustrate an example of medical device 100 to secure a catheter 1 (not shown in FIGS 1A-1C, but diagrammatically shown in FIG 3A-3B) to a patient's skin. The medical device is shown in three different positions. The position of FIG 1C is an intermediate position between the positions of FIGS 1A and 1B.

The medical device 100 comprises a pair of upper jaws and a pair of lower jaws. The pair of upper jaws comprises a left upper jaw 101 and a right upper jaw 102 adapted to receive a catheter therebetween. The pair of lower jaws comprises a left lower jaw 103 and a right lower jaw 104 adapted to receive the catheter therebetween. The medical device 100 also comprises a pair of guiding rods 109 connecting the left and right upper jaws 101, 102, and guiding the left and right upper jaws 101, 102 in translation relative to each other. The device further comprises a first pair of inclined rods 107 connecting the left upper jaw 101 to the right lower jaw 104 and a second pair of inclined rods 108 connecting the right upper jaw 102 to the left lower jaw 103 so that the pair of lower jaws 103, 104 closes when the pair of upper jaws 101, 102 opens and the pair of lower jaws 103, 104 opens when the pair of upper jaws closes 101, 102.

The inclined rods 107, 108 are rigid and non-articulated. They are made, for instance, by a metallic or plastic rod. The inclined rods 107, 108 rigidly connect the upper jaws to the lower jaws and allow the opposite movement of the pair of upper and lower jaws, i.e. the upper jaws 101, 102 close when the lower jaws 103, 104 open and vice versa. The maximum opening of the upper jaws 101, 102 is defined when the lower jaws 103, 104 are in contact with each other, and vice versa. The inclined rods 107 of the first pair do not cross each other and are sufficiently distant from each other to allow a catheter to be received between them. Similarly, the inclined rods 108 of the second pair do not cross each other and are sufficiently distant from each other to allow a catheter to be received between them.

The guiding rods 105,106 are rigid and non-articulated. They are made, for instance, by a metallic or plastic rod. The guiding rods 109 allow the left and right upper jaws 101, 102 to be moved in translation relative to each other and. Because of the inclined rods 107, 108, when the left and right upper jaws 101, 102 move in translation relative to each other, the left and right lower jaws 103, 104 also move in translation relative to each other. It is noteworthy that a constant distance D (see FIGS 3A and 3B) is kept between the pair of upper and lower jaws, i.e. the pair of upper jaws does not move toward or away from the pair of lower jaws.

FIG 1A illustrates the medical device 100 when the pair of upper jaws 101, 102 is opened (fully opened) and the pair of lower jaws 103, 104 is closed. FIG 1B illustrates the medical device 100 when the pair of upper jaws 101, 102 is closed and the pair of lower jaws 103, 104 is opened (fully opened). FIG 1C illustrates the medical device 100 in an intermediate position when the pair of upper jaws 101, 102 is partially opened and the pair of lower jaws 103, 104 is partially opened.

For securing a catheter to a patient's skin with the medical device 100, a catheter 1 is arranged between the pair of upper jaws 101, 102 and between the pair of lower jaws 103, 104 when the medical device 100 is in an intermediate position such as illustrated in FIG 1C. In the intermediate position, the upper jaws do not clamp the catheter and the lower jaws do not clamp the catheter either. Then, the pair of lower jaws 103, 104 closes so that the lower jaws 103, 104 clamp the catheter while the pair of upper jaws 101, 102 opens, as illustrated in FIGS 1A and 3A. The catheter 1 and the pair of lower jaws 103, 104 are inserted into the patient's body through the patient's skin 2, until the pair of upper jaws 101, 102 is close to or abuts against the outer surface 2A of the patient's skin 2. Finally, the pair of upper jaws 101, 102 are closed so that the upper jaws 101, 102 clamp the catheter 1 while the pair of lower jaws opens 103, 104, as illustrated FIG 1B and 3B.

As illustrated in FIGS 1A to 1C, the pair of upper jaws 101, 102 and the pair of lower jaws 103, 104 may form upper and lower rings when they close. The inner surfaces of the rings, or inner contact surfaces, are intended to be in contact with the catheter 1 when the upper jaws and/or the lower jaws close. In the example, the inner contact surface of the upper and lower rings are each provided with two diametrically opposed teeth 111 for gripping the catheter 1 when the upper jaws close. Thus, the catheter 1 is gripped more efficiently. The teeth 111 are an example of protrusions within the meaning of the invention.

Two press buttons 112 are respectively provided on the upper jaws 101, 102, for instance, in diametrically opposed positions. The press buttons 112 form large surfaces (compared to the relatively thin outer rim of the upper jaws) on which a pressure can be exerted easily, for instance with the thumb and the index finger, in order to close the pair of upper jaws.

As illustrated in FIGS 1A to 1C and already mentioned, the medical device 100 comprises a pair of guiding rods 105, 106 connecting the left and right upper jaws 101, 102, and guiding them in translation, in a translation direction, relative to each other. The guiding rods 105, 106 are parallel, extend in the translation direction and are sufficiently distant from each to allow the catheter 1 to pass between them. Each upper jaw is provided with two through holes 114 extending in the direction of the translation for receiving the guiding rods, respectively. The guiding rods 105, 106 pass through the holes 114 and can slide into the holes 114, in the translation direction. As a result, the left and right upper jaws 101, 102 can translate relative to each other. As illustrated in FIG 1A, when the pair of upper jaws 101, 102 is open, the ends of the guiding rods 105, 106 do not protrude, or slightly protrude, outside the outer rim of the upper jaws 101, 102. The ends of the guiding rods 105, 106 protrude more when the pair of upper jaws 101, 102 is closed, as illustrated in FIG 1B. In the example, the ends of the guiding rods 105, 106 are provided with stoppers 110 so as to prevent the guiding rods from separating from the upper jaws 101, 102.

In the example, the guiding rods 105, 106 are each provided with one spring 109 which pushes the right upper jaw 103 away from the left upper jaw 102. The springs 109 are compression springs. Providing two springs 109 allows a better force distribution but only one spring 109 could be used. In the example, the springs 109 are disposed around the guiding rods 105, 106. The opposite ends of each spring 109 abut, respectively, against the upper jaws 101, 102.

The medical device 100 may comprise a locking mechanism for locking the pair of upper jaws 101, 102 in their closed position. Consequently, the locking mechanism locks the pair of lower jaws 103, 104 in their open position.

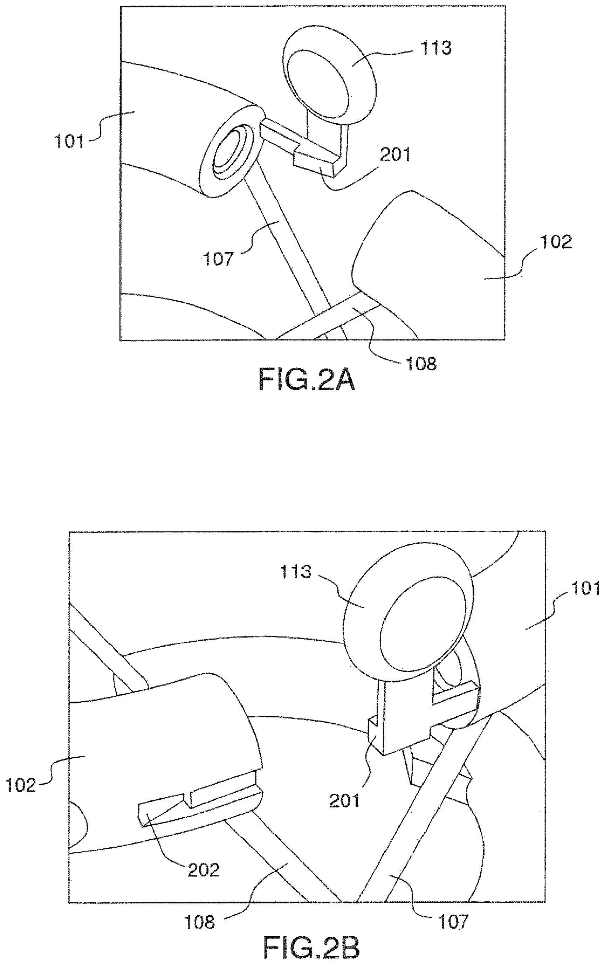

FIGS 2A and 2B are detailed views of FIGS 1A to 1C illustrating an example of a locking mechanism. The locking mechanism comprises at least one hook 201 provided on the upper jaw 101 and a locking notch 202 provided on the other upper jaw 102, the hook 201 interlocking with the locking notch 202 when the upper jaws 101, 102 are closed. The locking mechanism may comprise a locking or unlocking button connected to the hook or to the locking notch. In the example, the unlocking button 113 is rigidly connected to the hook 201, so that the hook 201 is pulled out of the notch 202 by pulling on the unlocking button 113. Once the hook 201 is unlocked, the upper jaws 101, 102 automatically open due to the force exerted by the springs 109.

FIGS 3A and 3B are schemes of steps of the method for securing a catheter 1 to a patient's skin 2 using a medical device 100 such as that of FIG 1A. The method may comprise a first step (not illustrated) of arranging the catheter 1 between the pair of upper jaws 101, 102 and between the pair of lower jaws 103, 104, while the medical device is in the intermediate position illustrated in FIG 1C. The next step may be closing the pair of lower jaws 103, 104 so that the lower jaws 103, 104 clamp the catheter 1 while the pair of upper jaws 101, 102 open, as illustrated in FIG 3A. Then the catheter 1 and the pair of lower jaws 103, 104 are inserted into the patient's body, through the patient's skin 2, until the pair of upper jaws 101, 102 abuts against the outer surface 2A of the patient's skin 2, as shown in dotted line in FIG 3A. Finally, as illustrated in FIG 3B, the pair of upper jaws 101, 102 are closed so that the upper jaws 101, 102 clamp the catheter 1, thus preventing the relative movement between the medical device 100 and the catheter 1. While the upper jaws 101, 102 close, the lower jaws 103, 104 open. The lower jaws 103, 104 open under the outer surface 2A of the patient's skin 2 and, thus, the lower jaws enter the patient's tissues as diagrammatically illustrated in FIG 3B. As a result, the medical device 100 is anchored in the patient's tissues and the relative movement between the medical device 100 (and thus the catheter 1) and the patient's skin is prevented. In other terms, the catheter 1 is fixed in place. Moreover, as illustrated FIG 3A and 3B, such a device makes it possible to secure the catheter approximatively perpendicular to the patient's skin.

For mobilizing a catheter 1 already fixed in place by the medical device 100, the pair of upper jaws 101, 102 is partially opened so that the upper jaws 101, 102 do not clamp the catheter 1 anymore and the lower jaws 103, 104 do not clamp the catheter 1 either. Thus, while the medical device 100 is held in place, the catheter 1 can slide inside the medical device 100 and be moved (inserted or withdrawn) relative to the patient's body. Once the catheter 1 is placed in the new desired position, the pair of upper jaws 101, 102 is closed so that the upper jaws 101, 102 clamp the catheter 1 and the lower jaws 103, 104 open, thereby fixing the catheter 1 in place.

While only some selected embodiments have been chosen to illustrate the present invention, it will be apparent to those skilled in the art from this disclosure that various changes and modifications can be made herein without departing from the scope of the invention as defined in the appended claims. For example, the size, shape, location or orientation of the various components can be changed as needed and/or desired. The structures and functions of one embodiment can be adopted in another embodiment. Further, it is not necessary for all advantages to be present in particular embodiments at the same time.

## Claims

1. A medical device to secure a catheter to a patient's skin comprising:
a pair of upper jaws comprising a left upper jaw (101) and a right upper jaw (102) adapted to receive a catheter (1) therebetween;
a pair of lower jaws comprising a left lower jaw (103) and a right lower jaw (104) adapted to receive the catheter therebetween;
a pair of guiding rods (105, 106) connecting the left and right upper jaws (101, 102), and guiding the left and right upper jaws (101, 102) in translation relative to each other; and
a first pair of inclined rods (107) connecting the left upper jaw (101) to the right lower jaw (104) and a second pair of inclined rods (108) connecting the right upper jaw (102) to the left lower jaw (103), so that the pair of lower jaws (103, 104) closes when the pair of upper jaws (101, 102) opens and the pair of lower jaws (103, 104) opens when the pair of upper jaws (101, 102) closes.

2. The medical device of claim 1, wherein the pair of upper jaws (101, 102) and lower jaws (103, 104) respectively form upper and lower rings when they are closed, each jaw having a half-circle shape.

3. The medical device of claim 2, wherein the diameter of the upper ring is similar to the diameter of the lower ring.

4. The medical device according to any of claims 1 to 3, wherein the inclined rods of the first pair of inclined rods (107) are parallel and the inclined rods of the second pair of inclined rods (108) are parallel.

5. The medical device according to any of claims 1 to 4, wherein the pair of guiding rods (105, 106) is provided with at least one spring (109) which pushes the right upper jaw (102) away from the left upper jaw (101).

6. The medical device according to any of claims 1 to 5, comprising a locking system for locking the pair of upper jaws (101, 102) in a closed position.

7. The medical device according to claim 6, wherein the locking mechanism comprises at least one hook (201) provided on one of the upper jaws and a locking notch (202) provided on the other upper jaw, the hook (201) interlocking with the locking notch (202) when the pair of upper jaws (101, 102) is closed.

8. The medical device according to any of claims 1 to 7, wherein the locking mechanism may comprise a locking or unlocking button (113) connected to the hook or the locking notch.

9. The medical device according to any of claims 1 to 8, wherein the guiding rods (105, 106) go through the upper jaws and the ends of the guiding rods (105, 106) are provided with a stopper (110) such that the relative movement between a guiding rod and an upper jaw is limited when the jaw abuts against the stopper.

10. The medical device according to of any claims 1 to 8, wherein each guiding rod (105, 106) is telescopic.

11. The medical device according to any of claims 1 to 10, wherein the upper jaws (101, 102) and/or the lower jaws (103, 104) define an inner contact surface intended to be in contact with the catheter when the upper jaws and/or the lower jaws close, the inner contact surface being provided with at least one protrusion (111) for gripping the catheter when the upper jaws and/or the lower jaws close.

12. The medical device according to any of claims 1 to 11, wherein two press buttons (112) are respectively provided on the upper jaws (101, 102) in diametrically opposed positions.

13. A method for securing a catheter to a patient's skin using the medical device according to any of claims 1 to 12.

14. The method according to claim 13, comprising the steps of:
arranging the catheter between the pair of lower jaws (103, 104) and between the pair of upper jaws (101, 102);
closing the pair of lower jaws so that the lower jaws (103,104) clamp the catheter while the pair of upper jaws (101,102) are opened;
inserting the catheter and the pair of lower jaws into the patient's body, through the patient's skin, until the pair of upper jaws (101, 102) are close to or abuts against the outer surface of the patient's skin; and
closing the pair of upper jaws (101, 102) so that the upper jaws clamp the catheter while the pair of lower jaws (103, 104) opens, thereby fixing the medical device and catheter in position.

15. A method according to claim 13 or 14, further comprising the steps of:
partially opening the pair of upper jaws so that the upper jaws (101, 102) do not clamp the catheter anymore and the lower jaws do not clamp the catheter (1) either;
moving the catheter (1) relative to the medical device; and
closing the pair of upper jaws (101, 102) so that the upper jaws clamp the catheter (1) and the lower jaws (103, 104) open.
